# EUROPEAN PATENT SPECIFICATION

(11) **EP 0 743 934 B1**
(45) Date of publication and mention of the grant of the patent: **03.11.1999**
(21) Application number: 95922074.0
(22) Date of filing: 06.02.1995
(51) Int. Cl.: C07C 17/386, C07C 19/08

(54) **PROCESS FOR SEPARATING PENTAFLUOROETHANE FROM A MIXTURE COMPRISING HALOGENATED HYDROCARBONS AND CHLOROPENTAFLUOROETHANE**
VERFAHREN ZUR TRENNUNG VON PENTAFLUORETHAN AUS EINER MISCHUNG, DIE HALOGENIERTE KOHLENWASSERSTOFFE UND CHLORPENTAFLUORETHAN ENTHÄLT
PROCEDE DE SEPARATION DE PENTAFLUOROETHANE D'UN MELANGE COMPORTANT DES HYDROCARBURES HALOGENES ET DU CHLOROPENTAFLUOROETHANE

(30) Priority: 07.02.1994 US 192664; 01.02.1995 US 378349
(43) Date of publication of application: 27.11.1996
(73) Proprietor: E.I. DU PONT DE NEMOURS AND COMPANY, Wilmington Delaware 19898 (US)
(72) Inventor: MAHLER, Barry, Asher, Glen Mills, PA 19342-1258 (US); MILLER, Ralph, Newton, Newark, DE 19711-2477 (US)
(74) Representative: Woodcraft, David Charles
(86) International application number: US9501191
(87) International publication number: WO9521148

(56) References cited:
- EP-A- 0 626 362
- CH-A- 471 760

## Description

The instant invention relates to a process for separating pentafluoroethane from a mixture comprising pentafluoroethane and at least one of chloropentafluoroethane and trifluoroethane.

### BACKGROUND OF THE INVENTION

New regulations have been designed to protect the stratospheric ozone layer from possible damage by fully halogenated chlorofluorocarbons. Pentafluoroethane (CHF₂CF₃ or HFC-125) is a valuable non-chlorine containing fluorocarbon that is especially valuable as a refrigerant, blowing agent, propellant, fire extinguishing agent, sterilant carrier gas, among other uses.

Pentafluoroethane may be prepared by chlorofluorinating perchloroethylene (perclene or CCl₂CCl₂) to produce a mixture comprising trichlorotrifluoroethane (CF₂ClCFCl₂ or CFC-113), dichlorotetrafluoroethane (CF₂ClCF₂Cl or CFC-114) and dichlorotrifluoroethane (CFCl₂CHF₂ or HCFC-123), and, after removing trichlorotrifluoroethane, fluorinating the remaining mixture to produce a mixture comprising pentafluoroethane (HFC-125), chloropentafluoroethane (CF₃CF₂Cl or CFC-115), and smaller amounts of other fluorinated compounds, e.g., 1,1,1-trifluoroethane (CF₃CH₃ or HFC-143a). Such a chlorofluorinating method is described in U.S. Patent No. 3,755,477. Various other methods for making pentafluoroethane together with chloropentafluoroethane are known and are described, for example, in U.S. Patent Nos. 3,518,550, 3,258,500, Japanese Patent Application Publication Nos. JP 03/099026, JP 04/029941, European Patent Application Publication No. 0 506 525, and World Intellectual Property Organization Publication No. WO 91/05752.

Whenever an extractive distillation process is employed for separating certain compounds, it is necessary to discover an extractive agent which will be effective to sufficiently enhance the desired separation process.

Extractive distillation agents that are capable of separating pentafluoroethane from chloropentafluoroethane are disclosed in U.S. Patent No. 5,087,329 in which chlorofluorocarbons such as dichlorotetrafluoroethane (CFC-114) are employed. However, these CFC compounds are relatively expensive, and regulations concerning protection of the ozone layer will likely cause CFC compounds to be phased out as commercial products thereby making CFCs unavailable or uneconomic to use.

### SUMMARY OF THE INVENTION

In a first aspect of the invention there is provided a process for separating pentafluoroethane from a first mixture comprising pentafluoroethane and at least one of 1,1,1-trifluoroethane and a chloropentafluoroethane, the process comprising the steps of:
adding at least one extractive agent comprised of an aliphatic alcohol or mixture of aliphatic alcohols having a boiling point at atmospheric pressure greater than about 64 degrees C and less than about 100 degrees C, to the first mixture in order to form a second mixture;
separating at least one of chloropentafluoroethane and 1,1,1-trifluoroethane from the second mixture by extractively distilling the second mixture in an extractive distillation zone and thereby separating said at least one of chloropentafluoroethane and 1,1,1-trifluoroethane and forming a third mixture comprising the extractive agent and pentafluoroethane and optionally 1,1,1-trifluoroethane; and
separating the pentafluoroethane from the third mixture to yield pentafluoroethane.

In a second aspect of the invention there is provided a process for separating 1,1,1-trifluoroethane from a first mixture comprising 1,1,1-trifluoroethane and pentafluoroethane, the process comprising the steps of :
adding at least one alcohol containing extractive agent having a boiling point at atmospheric pressure greater than about 64 degrees C, wherein said agent is substantially fluorine-free, to the first mixture in order to form a second mixture;
separating 1,1,1-trifluoroethane and pentafluoroethane by extractively distilling the second mixture in an extractive distillation zone and thereby separating 1,1,1-trifluoroethane and pentafluoroethane; and,
recovering at least one product from the group of 1,1,1-trifluoroethane and pentafluoroethane.

In a third aspect of the invention there is provided a process for separating a chlorofluorocarbon and 1,1,1-trifluoroethane from a first mixture comprising a chlorofluorocarbon, 1,1,1-trifluoroethane and pentafluoroethane, the process comprising the steps of:
adding at least one alcohol extractive agent having 1 to 4 carbon atoms and having a boiling point at atmospheric pressure of at least about 64°C to the first mixture in order to form a second mixture;
separating said at least one of chlorofluorocarbon and 1,1,1-trifluoroethane from the pentafluoroethane of the second mixture by extractively distilling the second mixture in an extractive distillation zone and thereby recovering said at least one of chlorofluorocarbon and 1,1,1-trifluoroethane as an overhead stream and forming a third mixture comprising the extractive agent and pentafluoroethane as a bottoms stream; and.
separating the extractive agent from the third mixture to yield a product from the group consisting of pentafluoroethane and pentafluoroethane with 1,1,1-trifluoroethane.

Conventionally used methods for manufacturing pentafluoroethane typically produce a mixture comprising pentafluoroethane (HFC-125) and chloropentafluoroethane (CFC-115). Such a mixture can form an azeotropic or azeotrope-like composition. The normal boiling points of pentafluoroethane and chloropentafluoroethane are relatively close, i.e., about -48.5 degrees C for pentafluoroethane and about -38.7 degrees C for chloropentafluoroethane, and their relative volatility is below about 1.1 at concentrations of pentafluoroethane greater than about 87.5 mole %, and below about 1.01 at concentrations greater than about 95 mole %. The above boiling points and relative volatilities indicate that it would be extremely difficult, if not impossible, to recover substantially pure pentafluoroethane from such a mixture by conventional distillation. By "conventional distillation" it is meant that the relative volatility only of the components of the mixture to be separated is being used to separate certain compounds within the mixture.

An alternate method of separating specific components in a mixture is by extractive distillation. Extractive distillation is employed when the components of the mixture may have differing volatilities, but such a difference is insufficient to permit effective separation of the components by using "conventional distillation". In conventional extractive distillation, an extractive agent is added that causes the difference in volatilities of the components in the starting mixture to be amplified such that the relative volatility becomes sufficient to permit separation of the components. By "conventional extractive distillation", it is meant that the components which have the higher and lower volatilities in the absence of the extractant retain their relative volatilities respectively when in the presence of the extractant; but the difference in the volatilities between the components has increased. In such a "conventional extractive distillation", HFC-125, which has the higher volatility versus CFC-115 in the absence of an extractive agent, would still have the higher volatility in the presence of CFC-115 and the extractive agent. In such a "conventional extractive distillation", CFC-115 would be removed along with the extractive agent from the bottom of the distillation column, and HFC-125 would be recovered in an overhead product stream.

It was surprising and unexpected that the instant invention can successfully employ simple alcohols such as methanol and ethanol as extractive distillation agents for separating pentafluoroethane from chloropentafluoroethane. Even more surprising was the manner in which the inventive extractive agent(s) functions, because HFC-125, which in the absence of the extractive agent(s) of the invention has the higher volatility versus that of CFC-115, has the lower volatility versus CFC-115 when in the presence of the extractive agent(s) of the invention. Specifically, the extractive agent of the invention surprisingly causes the expected or predicted relative volatilities of HFC-125 and CFC-115 to be reversed, wherein HFC-125 is removed along with the extractive agent from the bottom of the distillation column, and CFC-115 can be recovered in an overhead product stream.

The instant invention solves the problems associated with conventional distillation methods by employing extractive distillation processes as described above; in particular by a process for separating pentafluoroethane from a first mixture comprising pentafluoroethane and at least one of chloropentafluoroethane and 1,1,1-trifluorethane (HFC-143a), and optionally 1,1,1,2-tetrafluoroethane (HFC-134a) and hydrochlorofluorocarbons (HCFCs) such as chlorodifluoromethane (HCFC-22), HCFC-124, 124a, among others, which comprises:
(i) adding an extractive distillation agent comprising an alcohol having an atmospheric boiling point greater than about 64 degrees C and less than about 100 degrees C, to the first mixture in order to form a second mixture;
(ii) separating pentafluoroethane, for example, from chloropentafluoroethane and trifluoroethane of the second mixture by extractively distilling the second mixture in an extractive distillation zone thereby recovering as an overhead product a chloropentafluoroethane and 1,1,1-trifluoroethane containing stream that is substantially free of pentafluoroethane, and recovering a third mixture comprising the extractive agent and pentafluoroethane that is substantially free of chloropentafluoroethane and 1,1,1-trifluoroethane from the bottom region of a distillation column; and,
(iii) separating the pentafluoroethane from the extractive agent in the third mixture to yield a pentafluoroethane product.

By "substantially free" it is meant that the HFC-125 contains less than 1.0 wt% CFC-115 and/ or HFC-143a, normally less than 0.05 wt% CFC-115 and/or HFC-143a and in some cases less than about 10 ppm by weight CFC-115 and/or HFC-143a.

The first mixture can be obtained from any suitable manufacturing process or source. For example, the first mixture can be obtained as a product stream from the process disclosed in WIPO Publication No. WO 92/19576.

The invention is carried out in a manner such that in the presence of the extractive agent the volatility of the normally higher boiling chloropentafluoro-ethane, and in some cases HFC-143a, is enhanced relative to the normally lower boiling pentafluoroethane. By enhancing the volatility of the chloropentafluoro-ethane and/or HFC-143a in comparison to the pentafluoroethane, the chloropenta-fluoroethane and/or HFC-143a surprisingly becomes more volatile thereby permitting chloropentafluoroethane and/or HFC-143a to be removed from the top of the distillation column. In other words, the extractive agent(s) of the invention induce a reversal in the normal relative volatilities of CFC-115 and HFC-125 and/or of HFC-143a and HFC-125 such that the extractant and HFC-125 are removed from the bottom of the column. The relative volatility switch or reverse acting behavior is unexpected because it was heretofore impossible to use an extractive agent for reversing the predicted volatility of pentafluoroethane and chloropenta-fluoroethane, and of pentafluoroethane and HFC-143a.

The extractive agents used in the present invention are commercially available. If desired, the alcohol extractive agents of the invention can be removed from the HFC-125 by using conventional distillation methods. For example, an ethanol or a methanol extractive agent can be removed from HFC-125 and recycled to the distillation column for separating additional quantities of HFC-125 from CFC-115. Further, these alcohols are soluble in water so that even traces of the extractive agent can be readily removed from the HFC-125 by extracting or scrubbing with water.

Representative alcohols which are suitable for use as an extractive agent comprise or consist essentially of those containing 1 to 4 carbon atoms and having an atmospheric boiling point between about 64 and about 100 degrees C, e.g., aliphatic alcohols. Specific examples of such alcohols comprise or consist essentially of at least one member selected from the group of methanol, ethanol, n-propanol, isopropanol, sec-butanol, tert-butanol, among others.

We have found that 1,1,1-trifluoroethane (HFC-143a) can similarly be removed from pentafluoroethane concomitantly with CFC-115 or in a separate operation by using the inventive process. We have also found that CFC-115 can be selectively removed from a HFC-125 containing mixture comprising at least one of HFC-143a, HFC-134a, HCFC-22, among others.

Depending upon the quantity of HFC-143a, an azeotrope distillation can also be performed before and/or after the extractive distillation method. For example, a first mixture comprising pentafluoroethane and HFC-143a is introduced to a distillation column which is operated under conditions that cause formation of an azeotropic or azeotrope-like composition between HFC-143a and HFC-125. Such a composition can be removed from the distillation column thereby purifying the remaining HFC-125.

### BRIEF DESCRIPTION OF THE DRAWINGS

Figure 1 - FIG. 1 is a schematic diagram of an extractive distillation system which can be used for practicing the process of the invention.

Figure 2-FIG. 2 is a graphical representation of an azeotropic and azeotrope-like composition consisting essentially of HFC-125 and HFC-143a at a temperature of about -16.9 deg C.

### DETAILED DESCRIPTION

The pentafluoroethane and chloropentafluoroethane, which are typically the primary constituents of a first mixture, in their separated and pure state have normal boiling points of -48.5 and -38.7 degrees C, respectively. The relative volatility at atmospheric pressure of pentafluoroethane/chloropentafluoroethane was found to be nearly 1.0 as 100% purity of pentafluoroethane was approached. These data indicate that using conventional distillation procedures will not separate a substantially pure compound from the first mixture. By "substantially pure", it is meant that a given compound is at least about 99% pure on a weight basis.

To determine the relative volatility of pentafluoroethane and chloropentafluoroethane the so-called PTx Method was used. In this procedure, the total absolute pressure in a PTx cell of known volume is measured at a constant temperature for various known binary compositions. Use of the PTx Method is described in greater detail in "Phase Equilibrium in Process Design", Wiley-Interscience Publisher, 1970, written by Harold R. Null, on pages 124 to 126; the entire disclosure of which is hereby incorporated by reference.

These measurements can be reduced to equilibrium vapor and liquid compositions in the cell by an activity coefficient equation model, such as the Non-Random, Two-Liquid (NRTL) equation, to represent liquid phase non-idealities. Use of an activity coefficient equation, such as the NRTL equation, is described in greater detail in "The Properties of Gases and Liquids, 4th edition, publisher McGraw Hill, written by Reid, Prausnitz and Poling, on pages 241 to 387; and in "Phase Equilibria in Chemical Engineering, published by Butterworth Publishers, 1985, written by Stanley M. Walas, pages 165 to 244; the entire disclosure of each of the previously identified references are hereby incorporated by reference.

The result of the PTx measurements and the above series of calculations for a binary mixture containing HFC-125 and CFC-115 are summarized in Tables 1 through 3, giving results for -25 degrees C, 0 degrees C, and 25 degrees C. Similar measurements were obtained and similar calculations were performed for other binary combinations of compounds that relate to the invention.

Without wishing to be bound by any theory or explanation, it is believed that the NRTL equation can sufficiently predict whether or not pentafluoroethane and chloropentafluoroethane mixtures and/or the following other mixtures behave in an ideal manner, and can sufficiently predict the relative volatilities of the components in such mixtures.

**Table 1**

| Vapor-Liquid Measurements on the HFC-125/CFC-115 System at -25 deg. C | | | | |
|---|---|---|---|---|
| Mole % CFC-115 | | | Pressure Meas. (psia) | Relative Volatility HFC-125/CFC-115 |
| Charge | Liquid | Vapor | | |
| 17.67 | 17.72 | 15.85 | 40.75 | 1.144 |
| 13.48 | 13.51 | 12.38 | 40.75 | 1.105 |
| 10.40 | 10.42 | 9.74 | 40.95 | 1.078 |
| 7.40 | 7.42 | 7.08 | 41.10 | 1.052 |

**Table 2**

| Vapor-Liquid Measurements on the HFC-125/CFC-115 System at 0 deg. C | | | | |
|---|---|---|---|---|
| Mole % CFC-115 | | | Pressure Meas. (psia) | Relative Volatility HFC-125/CFC-115 |
| Charge | Liquid | Vapor | | |
| 17.67 | 17.78 | 15.92 | 96.95 | 1.142 |
| 13.48 | 13.55 | 12.37 | 97.20 | 1.110 |
| 10.40 | 10.45 | 9.69 | 98.00 | 1.087 |
| 7.40 | 7.43 | 7.00 | 98.00 | 1.066 |

**Table 3**

| Vapor-Liquid Measurements on the HFC-125/CFC-115 System at 25 deg. C | | | | |
|---|---|---|---|---|
| Mole % CFC-115 | | | Pressure Meas. (psia) | Relative Volatility HFC-125/CFC-115 |
| Charge | Liquid | Vapor | | |
| 17.67 | 17.87 | 16.36 | 198.60 | 1.113 |
| 13.48 | 13.61 | 12.65 | 200.25 | 1.087 |
| 10.40 | 10.49 | 9.88 | 200.75 | 1.069 |
| 7.40 | 7.45 | 7.11 | 201.00 | 1.052 |

The "Charge" column refers to the quantity of CFC-115 that is in a mixture of HFC-125 and CFC-115 which is introduced into the PTx Cell. The "Relative/Volatility" and Mole % CFC-115 in the Vapor and Liquid were those calculated from the Pressure measured at the temperature indicated on the above Tables.

While the relative volatility of CFC-115 in comparison to HFC-125 at relatively low concentrations is sufficient to permit separating CFC-115 by using conventional distillation methods, the relative volatility is nearly 1.0 as 100% purity of HFC-125 as approached. A relative volatility approaching 1.0 would render removing low concentrations of CFC-115 from HFC-125 by using conventional distillation difficult if not impossible, e.g., conventional distillation would require using large and expensive distillation columns.

Using the equations developed for the above system at temperatures of about -25, 0 and about 25 degrees C, and when extrapolating to about -50 degrees C, the presence of a low-boiling azeotropic or azeotrope-like mixture is indicated, with a composition of about 10 mole % CFC-115 and 90 mole % HFC-125. The existence of such a mixture is described in greater detail in copending and commonly assigned U.S. Patent Application Serial No. 08/055,486, filed on April 30, 1993 and Serial No. 08/208,256, filed on March 9, 1994, both in the names of Mahler et al., and entitled "Process For Separating HCl And Halocarbons" (corresponding to WIPO Publication No. WO94/25419); the disclosure of which is incorporated by reference. For best results, the inventive extractive distillation process normally avoids using conditions that cause formation of the HFC-125/CFC-115 azeotropic or azeotrope-like compositions.

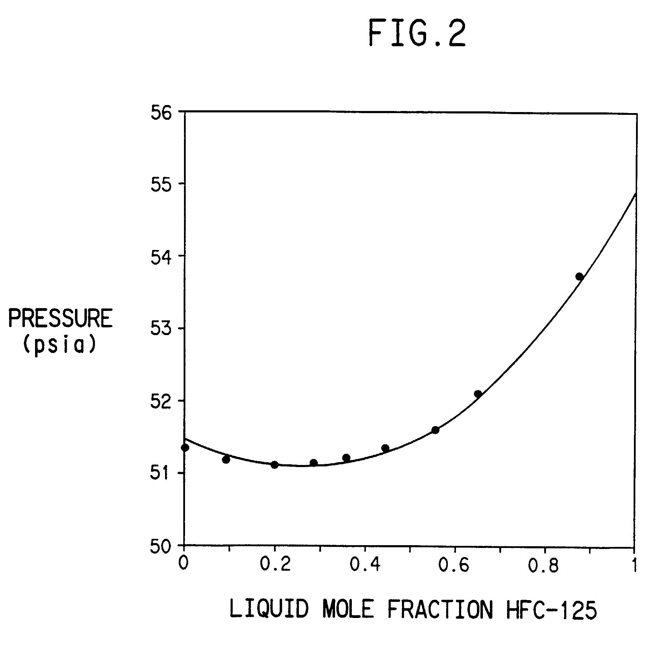

Similar difficulties occur when separating relatively small quantities of trifluoroethane (HFC-143a) from a pentafluoroethane (HFC-125) containing first mixture. The results of PTx measurements and calculations for the HFC-143a/HFC-125 system at -16.9 deg C can be summarized generally in Table 4 below (and shown graphically in Fig. 2 which is discussed below in greater detail).

**Table 4**

| Vapor-Liquid Measurements on the HFC-143a/HFC-125 System at -16.9 deg. C | | | |
|---|---|---|---|
| Mole % HFC-125 | | Pressure | Relative Volatility |
| Liquid | Vapor | (psia) | HFC-125/HFC-143a |
| 0.00 | 0.00 | 51.35 | 0.960 |
| 9.25 | 9.01 | 51.20 | 0.972 |
| 19.91 | 19.75 | 51.10 | 0.990 |
| 28.17 | 28.31 | 51.12 | 1.007 |
| 35.77 | 36.31 | 51.18 | 1.024 |
| 43.73 | 44.77 | 51.33 | 1.043 |
| 55.13 | 56.86 | 51.62 | 1.073 |
| 64.29 | 64.41 | 52.10 | 1.098 |
| 87.36 | 88.95 | 53.75 | 1.165 |
| 100.00 | 100.00 | 55.02 | 1.203 |

The relative volatility of HFC-125 in comparison to HFC-143a at relatively low concentrations of HFC-143a is sufficient to permit separating HFC-143a from HFC-125 by using conventional distillation methods, e.g., the relative volatility of HFC-125 and HFC-143a at low HFC-143a concentrations is much greater than 1.0. However, the relative volatility drops to 1.0 as HFC-125 concentrations approach about 20 mole %, thereby indicating the formation of an azeotropic or azeotrope-like composition. The formation of an azeotropic or azeotrope-like composition is also indicated by mixtures of HFC-143a and HFC-125 which at a given temperature have a lower vapor pressure than either pure component or the other HFC-125/HFC-143a mixtures at the given temperature, e.g., refer to Fig. 2. Such azeotropic or azeotrope-like formation would make it virtually impossible for conventional methods to produce HFC-125 that is substantially free of HFC-143a from an HFC-125 mixture which contains more than about 80 mole % HFC-143a. We have found that azeotropic or azeotrope-like compositions form between HFC-125 and HFC-143a at a variety of temperatures and pressures. For example, an azeotropic or azeotrope-like composition may be formed between a mixture consisting essentially of about 40 to about 95 mole % HFC-125 and about 60 to about 74 mole % HFC-143a when at a temperature of about -50 to about 10 deg C and a vapor pressure of about 12 to about 122 psia.

Whenever used in the specification and appended claims the terms below are intended to have the following definitions.

By "azeotrope" or "azeotropic" composition is meant a constant boiling liquid admixture of two or more substances that behave as a single substance. One way to characterize an azeotropic compositions or mixture is that the vapor produced by partial evaporation or distillation of the liquid has the same composition as the liquid from which it was evaporated or distilled, e.g., the admixture distills/refluxes without compositional change. Constant boiling compositions are characterized as azeotropic because they exhibit either a maximum or minimum boiling point, as compared with that of the non-azeotropic mixtures of the same components. A azeotropic composition can also be characterized as the maximum or minimum vapor pressure for a mixture at a given temperature when plotted as a function of liquid mole fraction.

By "azeotrope-like" composition is meant a constant boiling, or substantially constant boiling, liquid admixture of two or more substances that behaves as a single substance. One way to characterize an azeotrope-like composition is that the vapor produced by partial evaporation or distillation of the liquid has substantially the same compositions as the liquid from which it was evaporated or distilled, e.g., the admixture distills/refluxes without substantial compositional change. An azeotrope-like composition can also be characterized by the area, which is shown by plotting vapor pressure at given temperature as a function of liquid mole fraction, that is adjacent to the maximum or minimum vapor pressure.

Typically, a composition is azeotrope-like, if, after about 50 weight % of the composition is removed such as by evaporation or boiling off, the change between the original composition and the composition remaining is less than about 6% and normally less than about 3% relative to the original composition.

By "effective" amount is intended to refer to the amount of each component of the inventive compositions which, when combined, results in the formation of an azeotropic or azeotrope-like composition. This definition includes the amounts of each component, which amounts may vary depending on the pressure applied to the composition so long as the azeotropic or azeotrope-like compositions continue to exist at the different pressures, but with possible different boiling points. Effective amount also includes the amounts, such as may be expressed in weight percentages, of each component of the compositions of the instant invention which form azeotropic or azeotrope-like compositions at temperatures or pressures other than described herein. Therefore, included in this invention are azeotropic or azeotrope-like compositions consisting essentially of effective amounts of at least one of HFC-125 and HFC-143a such that after about 50 weight percent of an original composition is evaporated or boiled off to produce a remaining composition, the change between the original composition and the remaining composition is typically no more than about 6% and normally no more than about 3% or less relative to the original composition.

It is possible to characterize, in effect, a constant boiling admixture which may appear under many guises, depending upon the conditions chosen, by several criteria:
* The composition can be defined as an azeotrope of HFC-125 ("A") and HFC-143a ("B"), among others, because the term "azeotrope" is at once both definitive and limitative, and requires effective amounts of A,B for this unique composition of matter which can be a constant boiling composition.
* It is well known by those skilled in the art, that, at different pressures, the composition of a given azeotrope will vary at least to a degree, and changes in pressure will also change, at least to some degree, the boiling point temperature. Thus, an azeotrope of HFC-125 ("A") and HFC-143a ("B"), among others, represents a unique type of relationship but with a variable composition which depends on temperature and/or pressure. Therefore, compositional ranges, rather than fixed compositions, are often used to define azeotropes.
* The composition can be defined as a particular weight percent relationship or mole percent relationship of HFC-125("A") and HFC-143a ("B"), among others, while recognizing that such specific values point out only one particular relationship and that in actuality, a series of such relationships, presented by A,B actually exist for a given azeotrope, varied by the influence of pressure and/or temperature.
* An azeotrope of HFC-125("A") and HFC-143a ("B"), among others, can be characterized by defining the compositions as an azeotrope characterized by a boiling point at a given pressure, thus given identifying characteristics without unduly limiting the scope of the invention by a specific numerical composition, which is limited by and is only as accurate as the analytical equipment available.

The azeotrope or azeotrope-like compositions or the present invention may be formed by operating a conventional distillation apparatus, when practicing the inventive extractive distillation method, combining an effective amounts of the components by any convenient method including mixing, combining, among others. For example, one method is to weigh the desired components amounts, and thereafter combine them in an appropriate container.

Conventional distillation methods may be capable of separating an HFC-125 and HFC-143a containing mixture that includes more than the azeotropic or azeotrope-like concentration of HFC-125, when such methods are performed at a specific temperature and pressure. For example, substantially pure HFC-125 can be produced by distilling a mixture having more than about 20 mole % HFC-125 at a temperature of -16.9 deg c, thereby forming an azeotropic or azeotrope-like composition which is removed from the bottom of the distillation column. Substantially pure HFC-125 is recovered from the distillation column as an overhead product. In other words, forming the HFC-125/HFC-143a azeotropic or azeotrope-like composition may permit distilling a mixture comprising HFC-125 and HFC-143a and removing the HFC-143a as an HFC-125/HFC-143a azeotrope. By removing HFC-143a as a component of an azeotropic or azeotrope-like composition, substantially pure HFC-125 may be obtained, e.g., 99.99% by weight HFC-125.

HFC-125 and HFC-143a can each be purified by using the above-identified azeotropic and azeotrope-like compositions in a process that comprises using these azeotropic and azeotrope-like compositions in a conventional distillation method. For example, the conventional distillation column is operated at a temperature and pressure which causes these azeotropes to form. The azeotropic or azeotrope-like composition has a maximum boiling point, and the composition will be collected in the column bottoms. For example, where HFC-125 is present in excess of its azeotrope composition with HFC-143a, an azeotrope or azeotrope-like composition consisting essentially of HFC-125 and HFC-143a is collected in the column bottoms, thereby leaving substantially pure HFC-125 overhead as an overhead product. Conversely, where HFC-143a is present in excess of its azeotrope compositions with HFC-125, an azeotrope or azeotrope-like composition consisting essentially of HFC-125 and HFC-143a is collected in the column bottoms, leaving substantially pure HFC-143a as an overhead product.

When performing the previously described azeotrope distillation methods, a significant amount of the HFC-125 can remain within the azeotropic or azeotrope-like composition, such amount is dependent upon the amount of HFC-143a in the starting mixture. The quantity of HFC-125 remaining in the azeotropic or azeotrope-like composition is a function of the amount of HFC-125 relative to the HFC-143a in the starting or first mixture, e.g., the quantity of HFC-125 remaining in the azeotrope increases with increased amounts of HFC-143a in the starting mixture. The azeotropic or azeotrope-like composition, if desired, can be recovered as a useful product, e.g., for use as a refrigerant, blowing agent, and other uses. In the event that it is preferable to obtain either substantially pure HFC-143a or HFC-125, then the extractive distillation method of this invention should be practiced, i.e., the relative volatility for such compositions is sufficiently close to 1.0 that an impractically large distillation column would otherwise be required for separation using conventional distillation.

While the azeotropic distillation methods discussed above are useful, such conventional distillation methods are normally insufficient to recover substantially pure HFC-125 and/or HFC-143a at high yields and in a cost effective manner.

The problems associated with conventional distillation methods can be overcome by using the inventive extractive distillation method. Extractive distillation typically employs certain extractive agents to increase the relative volatility of the compound(s) to be separated; however, in the present invention the relative volatility of such compounds is altered in a manner that induces a reversal in the normal relative volatility. In other words, by adding the extractive agent(s) of the present invention, the volatility of CFC-115 and/or HFC-143a is increased much more than the volatility of HFC-125. Given the normal volatilities of pentafluoro-ethane and chloropentafluoroethane, and of pentafluoroethane and HFC-143a, it was also a surprising and an unexpected result that a mixture comprising pentafluoroethane and HFC-143a can be separated by an extractive distillation method that employs readily available and low cost alcohols. It was especially unexpected that the inventive process causes the normally higher boiling chloropentafluoroethane to form an overhead product in the distillation column whereas the pentafluoroethane and an alcohol extractant agent can be removed from the bottom of the column. Given the normal volatilities of pentafluoroethane and HFC-143a, it was a surprising and an unexpected result that a mixture comprising pentafluoroethane and trifluoroethane can be separated by an extractive distillation method that employs readily available and low cost alcohols. It was especially unexpected that the inventive process causes the normally higher boiling HFC-143a to form an overhead product in the distillation column whereas the pentafluoroethane and an alcohol extractant agent can be removed from the bottom of the column. Moreover, in one aspect of the invention, chloropentafluoroethane can be selectively removed from a mixture comprising pentafluoroethane, HFC-143a and chloropentafluoroethane, e.g., a substantially pure mixture consisting essentially of HFC-125 and HFC-143a can be produced.

Extractive distillation of the invention can be practiced by operating a continuous distillation system which comprises a multi-stage distillation column with a minimum of two feed points, a reboiler, an overhead condenser for returning reflux to the column, among other system components. For example, the extractive agent(s) can be introduced at a first feed point which is located above a second feed point that is used for introducing the mixture to be separated. The overhead product stream of the distillation column is removed at a point located above the first or second feed points, and the extractant is removed at a location below the first or second feed points.

In one aspect of the invention, an extractive agent, e.g., methanol, is introduced at an upper or the first feed point of the distillation column, whereas the mixture requiring separation, e.g., pentafluoroethane and at least one of chloro-pentafluoroethane and 1,1,1-trifluoroethane, is introduced at a relatively lower or the second feed point in the column. The liquid extractive agent passes downwardly through trays which are located in the center of the column. While in the presence of the extractive agent, chloropentafluoroethane becomes relatively more volatile than pentafluoroethane, thereby allowing chloropentafluoroethane that is substantially free of pentafluoroethane to exit the top of the column. The chloropentafluoroethane can then be condensed by using conventional reflux condensers. At least a portion of the condensed chloropentafluoroethane containing stream can be returned to the top of the column as reflux, and the remainder recovered as a substantially pure CFC-115, e.g., 98% by weight pure CFC-115. The ratio of condensed material, which is returned to the column, to the amount of material removed as product is commonly referred to as the "reflux ratio". The reflux ratio will define the physical characteristics of the distillation column. In general, an increase in the reflux ratio will in turn minimize the extractant concentration in the CFC-115 that is exiting overhead. Increasing the reflux ratio typically requires employing larger and more expensive distillation column.

While the CFC-115 is being recovered at the top of the distillation column, pentafluoroethane and an extractive agent are exiting the bottom of the column. The pentafluoroethane and extractive agent can then be separated by being passed through a stripper or distillation column that employs conventional distillation or other known methods. In some cases, at least a portion of the separated extractive agent can be recycled to the distillation column. The pentafluoroethane may then be readily isolated as a substantially pure product. Alternatively, the extractant can be removed from the HFC-125 by extracting or scrubbing with water.

The specific conditions which can be used for practicing the invention are interrelated and depend upon a number of design parameters such as the diameter of the column, selected feed points, the number of separation stages in the column, reflux ratio, among others. The operating pressure of the distillation system may range from about 15 to 350 psia, normally about 50 to about 300 psia. Typically, an increase in the extractant feed rate relative to the mixture to be separated will result in an increase in the purity of the recovered HFC-125. The temperature and heat transfer area of the overhead condenser is normally sufficient to substantially fully condense the CFC-115, or is optionally sufficient to achieve the desired reflux ratio by partial condensation.

The temperature that is employed at a given step in the inventive process is a function of the pressure and the design characteristics of the distillation column, e.g., reflux ratio. Typically, the temperature will range from about -25 to 200 C based upon an operating pressure of about 200 psia.

The quantity of CFC-115 in the first mixture, i.e., the HFC-125-containing mixture to be purified, can be reduced by using conventional distillation. While conventional distillation methods are normally incapable of producing HFC-125 that is substantially free of CFC-115, conventional distillation can be used for reducing the initial quantity of CFC-115 in the first mixture. For example, conventional distillation can be used for removing relatively large or bulk quantities of CFC-115 from the first mixture, which in turn is processed in accordance with the invention method, in order to obtain HFC-125 that is substantially free of CFC-115. Depending upon the quantity of HFC-125 in the first mixture, HFC-125 can be removed from a mixture containing relatively large quantities of CFC-115 thereby producing CFC-115 that is substantially free of HFC-125.

Similarly, the quantity of HFC-143a in the first mixture, i.e., the HFC-125-containing mixture to be purified, can be reduced by using conventional distillation. While conventional distillation methods are incapable of producing HFC-125 that is substantially free of HFC-143a, conventional distillation can be used for reducing the initial quantity of HFC-143a in the first mixture when the HFC-125 is present in excess of the azeotropic or azeotrope-like composition. For example, conventional distillation can be used for removing relatively large or bulk quantities of HFC-143a from the first mixture, which in turn is processed in accordance with the invention method, in order to obtain HFC-125 that is substantially free of HFC-143a. Depending upon the quantity of HFC-125 in the first mixture, HFC-125 can be removed from a mixture containing relatively large quantities of HFC-143a thereby producing HFC-143a that is substantially free of HFC-125.

The effective amount of the extractive agent(s) can vary widely. In general, however, using an increased amount of extractive agent will enhance the purity of the recovered CFC-115 and/or HFC-143a, (e.g., the removal rate of HFC-125 is increased) and the recovered HFC-125 (e.g., the removal rate of CFC-115 and/or HFC-143a is increased). Typically, the ratio of extractive agent to HFC-125 ranges from about 2 to about 10:1 on a weight basis, with the higher ratios necessary for the more difficult separations.

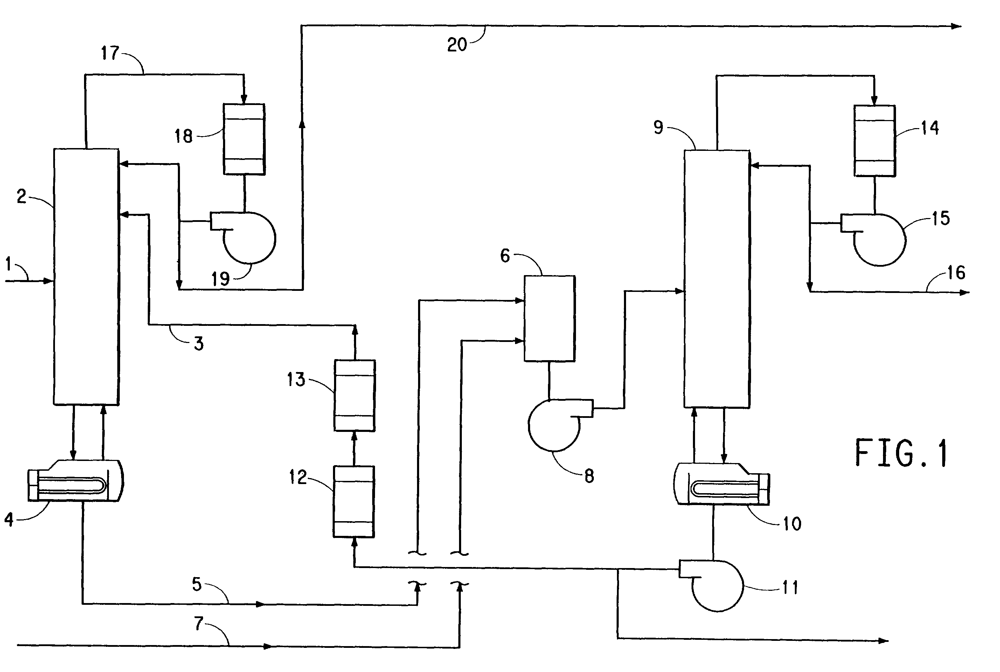

Referring now to the Figure, Fig. 1 schematically illustrates a system which can be used for operating the inventive extractive distillation process. A first mixture comprising HFC-125 and at least one of CFC-115, HFC-143a, HFC-134a, HCFC-22, HCFC-124, among others, is supplied via conduit 1 to extraction column 2. At least one liquid extractive agent is supplied via conduit 3 to the extraction column 2, and introduced into column 2 at a location above the mixture 1. A second mixture comprising the extractive agent, HFC-125 from the first mixture, and trace amounts of impurities such as HCFC-124 is removed from the column 2, and transported to steam heated reboiler 4. In some cases, the reboiler 4 is attached to the extractive column 2. The second mixture is supplied via conduit 5 to a feed tank 6. Supplemental liquid extractive agent is also supplied to feed tank 6 via conduit 7 thereby forming a third mixture or extraction agent recycle. A pump 8 transports the third mixture to a stripping column 9. Stripping column 9 separates the extractive agent from non-extractive agents. Extractive agent is removed from column 9 and supplied to a second steam heated reboiler 10. In some cases, the reboiler 10 is attached to column 9. Pump 11 transports the extractive agent from the reboiler 10 through a cold water chiller 12, and then to chiller 13. If necessary, excess quantities of extraction agent can be purged prior to reaching chiller 12. Typically, chiller 13 is operated at a temperature of about -25°C. After exiting chiller 13, the extraction agent is supplied via conduit 3 into extraction column 2.

The non-extractive agents or an HFC-125 containing stream exits from the top of stripping column 9 as an off gas, and are introduced into condenser 14, which is typically operated at a temperature of about -25°C. While under reflux conditions, pump 15 returns a portion of the HFC-125 to the stripping column 9. The remaining portion of the HFC-125 exits the system via conduit 16 as a high purity product, e.g., substantially pure HFC-125.

An off gas is also removed from extraction column 2, with the exception that the off gas from column 2 contains a CFC-115 and/or HFC-143a product which is substantially free from the HFC-125 of the first mixture. The CFC-115 and/or HFC-143a product is transported via conduit 17 to condenser 18. Condenser 18 is typically operated at a temperature of about -25°C. While under reflux conditions, pump 19 returns a portion of the CFC-115 and/or HFC-143a product to extraction column 2. A CFC-115 and/or HFC-143a product, which is substantially free of HFC-125, exits the system via conduit 20.

If desired the first mixture can be introduced into a third distillation column (not shown but equivalent in construction to column 2) prior to being introduced into column 2. The third distillation column can be used for removing bulk quantities of CFC-115 and/or HFC-143a. In some cases, such removal can be achieved by operating the third distillation column under conditions that form an azeotropic or azeotrope-like composition between HFC-143a and HFC-125.

The following examples are provided to illustrate certain aspects of the present invention; but not limit the scope of the invention as defined by the appended claims. Parts per million (ppm) concentrations are by weight unless otherwise specified. The following Examples employ the NRTL interaction parameters identified above. All Examples are based upon a feed rate of pentafluoroethane of about 1000 lb/hr, with the impurity content of chloropentafluoroethane or trifluoroethane at a level of either approximately 5 lb/hr (4975 ppm) or 50 lb/hr (4.8%). A different column design and operating conditions are used for the Examples in which extractive distillation is employed than those in which conventional distillation is employed, in order to enhance the performance of each distillation method.

In the following three Comparative Examples involving conventional distillation, the number of stages in the distillation column is either 101 or 121 stages, and the pentafluoroethane stream to be purified is fed onto column stage 50, with the column condenser corresponding to stage no. 1. Depending upon the desired product purity, the operating conditions are selected and various reflux ratios used to maximize the yield of such a product.

### Comparative Example 1

In this comparative example, conventional distillation is used in a column with 121 stages for purifying a feed stream containing 1000 lb/hr of pentafluoroethane (HFC-125) and only 5 lb/hr of chloropentafluoroethane (CFC-115), for a concentration of 4975 ppm. The feed is introduced at a temperature of -25 degrees C, with the column condenser pressure at 194.7 psia and the column base pressure 3 psi higher. The distillate temperature is 23.8 degrees C, and the bottom column temperature is 24.4 degrees C. Under these operating conditions, the product HFC-125 leaves in the overhead (distillate) stream from the column. The remaining HFC-125 exits in the bottoms stream with CFC-115 as a waste product. Conditions are set so that about 50% of the feed stream goes into the overhead and 50% to the bottoms.

**Table 5**

| Reflux Flow (lb/hr) | Reflux Ratio | Lb/hr HFC-125 in Overhead | ppm CFC-115 in Overhead | % HFC-125 Recovery |
|---|---|---|---|---|
| 100000 | 200/1 | 501 | 2116 | 50 |
| 200000 | 400/1 | 501 | 1936 | 50 |
| 300000 | 600/1 | 501 | 1871 | 50 |

The purity of the recovered HFC-125 is relatively low, even when a relatively large reflux ratio of 600/1 and a large number of distillation stages is employed.

### Comparative Example 2

In this comparative example, conventional distillation is used in a column with 101 stages for purifying a feed stream of the same composition as Comparative Example 1. The operating conditions are modified to take advantage of the CFC-115/HFC-125 azeotrope we previously found to exist at very low temperatures, taking the azeotrope overhead as a means of removing the small amount of CFC-115 in the feed. The feed is introduced at a temperature of -25 degrees C, with the column condenser pressure set at 19.7 psia and the column base pressure 3 psi higher. Under these conditions the distillate temperature is -42 degrees C, and the bottom column temperature is -38.8 degrees C. Under these operating conditions, the product HFC-125 leaves in the bottom stream from the column. An impurity level of 500 ppm CFC-115 is selected. The remaining HFC-125 is taken overhead with azeotroped CFC-115 as a waste product.

**Table 6**

| Reflux flow (lb/hr) | Reflux Ratio | Lb/hr HFC-125 in Bottoms | ppm CFC-115 in Bottoms | % HFC-125 Recovery |
|---|---|---|---|---|
| 10000 | 21/1 | 529 | 500 | 52.9 |
| 25000 | 329/1 | 930 | 500 | 93.0 |
| 50000 | 862/1 | 950 | 500 | 95.0 |
| 100000 | 1852/1 | 955 | 500 | 95.5 |

At a reflux ratio of 1852/1, and with the impurity level of 500 ppm CFC-115, the yield of HFC-125 product is less than about 95.5%.

### Comparative Example 3

In this comparative example, conditions are the same as Comparative Example 2 except that product purity requirement is increased: that is, the level of CFC-115 impurity allowed is reduced from 500 to 50 ppm.

**Table 7**

| Reflux flow (lb/hr) | Reflux Ratio | Lb/hr HFC-125 in Bottoms | ppm CFC-115 in Bottoms | % HFC-125 Recovery |
|---|---|---|---|---|
| 50000 | 115/1 | 565 | 50 | 56.5 |
| 100000 | 585/1 | 832 | 50 | 83.2 |
| 150000 | 1083/1 | 865 | 50 | 86.5 |

At a reflux ratio of 1083/1, about 15% of the HFC-125 is lost with the distillate.

The above examples show that conventional distillation is not an economical way to separate CFC-115 from HFC-125, when high purity HFC-125 is required, because such requires a relatively high reflux ratio, a large column diameter required to accommodate this high reflux ratio, high utilities cost for heating and cooling this large flow of material through the column, and the need for disposing of large quantities of waste material containing CFC-115. Such waste material will also contain a relatively large quantity of HFC-125.

In the following Examples involving extractive distillation, the distillation column has 57 or 42 stages, again with the column condenser counted as stage no. 1. The pentafluoroethane stream to be purified is introduced onto column stage 40, and the extractant is fed onto column stage 15 in the case of the 57 stage column, and onto stages 35 and 5 respectively in the case of the 42 stage column. In each Example, the column distillate/feed ratio (based on pentafluoro-ethane) is varied to achieve the desired product purity and recovery (yield) while the reflux ratio is fixed. In each example, the column pressure is 84.7 psia. In general, the column pressures need be chosen only so the temperature in the bottoms are below the critical temperatures of the individual components. Each of the Examples also includes a measurement of performance at varied levels of extractant flow rates.

### Example 1

In this example, extractive distillation is used in a column with only 57 theoretical stages for purifying a feed stream containing 1000 lb/hr of pentafluoro-ethane (HFC-125) and 5 lb/hr of chloropentafluoroethane (CFC-115) or 4975 ppm as in the Comparative Examples. The feed stream and methanol extractant are introduced at a temperature of 10 degrees C, with the column condenser pressure set at 84.7 psia and the column base pressure 3 psi higher. The methanol is fed at varying rates as shown in the table, while the reflux flow is held at 2,000 lb/hr. The distillate temperature is 8.0 degrees C, and the bottom column temperature varies from about 43.5 to 85.9 degrees C. The methanol and the HFC-125 exit through the bottom of the column, and the CFC-115 exits the top of the column.

In this and each example that follows, the methanol may be removed from the HFC-125 by using conventional distillation methods, e.g., a stripping column, or by scrubbing with water and drying with conventional methods such as a molecular sieve drier.

The compositions shown below in Table 7 are on a methanol-free basis.

**Table 8**

| Lb/hr Methanol Feed Rate | Lb/hr HFC-125 in Bottoms | ppm CFC-115 in Bottoms | % HFC-125 Recovery |
|---|---|---|---|
| 2000 | 999.9 | 2.9 | 99.99 |
| 3000 | 999.9 | 0.1 | 99.99 |
| 4000 | 999.9 | <0.1 | 99.99 |
| 5000 | 999.9 | <0.1 | 99.99 |

Example 1 shows that using methanol as an extractive agents permits recovering high purity HFC-125 at high yields while using a relatively small distillation column and extractant flow.

### Example 2

Example 2 is carried out in substantially the same way as Example 1 except that the feed stream contains 1000 lb/hr of pentafluoroethane (HFC-125) and 50 lb/hr of chloropentafluoroethane (CFC-115) or 4.8%. The distillate temperature is 8.4 degrees C, and the bottom column temperature varies from about 43.5 to 86.0 degrees C.

**Table 9**

| Lb/hr Methanol Feed Rate | Lb/hr HFC-125 in Bottoms | ppm CFC-115 in Bottoms | % HFC-125 Recovery |
|---|---|---|---|
| 2000 | 999.5 | 31 | 99.95 |
| 3000 | 999.5 | 1.1 | 99.95 |
| 4000 | 999.5 | 0.1 | 99.95 |
| 5000 | 999.5 | <0.1 | 99.95 |

Example 2 illustrates that high purity HFC-125 may be obtained at a high yield by using methanol as extractive distillation agent from a mixture having an impurity concentration (CFC-115) of about ten times greater than in the Comparative Examples.

### Example 3

Example 3 is carried out in substantially the same way as Example 1 except that the distillation column length is reduced from 57 to 42 stages. The distillate temperature is 8.0 degrees C, and the bottom column temperature ranges from 43.5 to 85.9 degrees C, depending on the flowrate of extractant.

**Table 10**

| Lb/hr Methanol Feed Rate | Lb/hr HFC-125 in Bottoms | ppm CFC-115 in Bottoms | % HFC-125 Recovery |
|---|---|---|---|
| 2000 | 999.9 | 78 | 99.99 |
| 3000 | 999.9 | 17 | 99.99 |
| 4000 | 999.9 | 6 | 99.99 |
| 5000 | 999.9 | 3 | 99.99 |

This Example demonstrates that a relatively short column can be employed by using a methanol extractive agent to yield high purity HFC-125.

### Example 4

Example 4 is carried out in substantially the same way as Example 1 except that ethanol is employed as the extractant. The distillate temperature is 2.9 degrees C, and the bottom column temperature ranges from 70.6 to 94.5 degrees C, depending on the flowrate of extractant.

**Table 11**

| Lb/hr Ethanol Feed Rate | Lb/hr HFC-125 in Bottoms | ppm CFC-115 in Bottoms | % HFC-125 Recovery |
|---|---|---|---|
| 5000 | 999.0 | 0.5 | 99.90 |
| 7500 | 999.0 | <0.1 | 99.90 |
| 10000 | 999.0 | <0.1 | 99.90 |

This Example demonstrates that ethanol is an effective extractive agent for separating CFC-115 and HFC-125.

### Example 5

Example 5 is carried out in substantially the same way as Example 1 except that the extractant used is n-propanol. The distillate temperature is 2.7 to 2.9 degrees C, and the bottom column temperature ranges from 54.6 to 85.2 degrees C, depending on the flowrate of extractant.

**Table 12**

| Lb/hr Propanol Feed Rate | Lb/hr HFC-125 in Bottoms | ppm CFC-115 in Bottoms | % HFC-125 Recovery |
|---|---|---|---|
| 5000 | 999.0 | 228 | 99.90 |
| 7500 | 999.0 | 21 | 99.90 |
| 10000 | 999.0 | 5 | 99.90 |

This Example demonstrates that propanol is an effective extractive agent for separating CFC-115 and HFC-125.

### Comparative Example 4

In this example, the goal is to obtain a HFC-125 product having 100 ppm or less of HFC-143a. Conventional distillation is used in a column with 62 stages for purifying a feed stream containing 1000 pph of pentafluoroethane (HFC-125) and 5 pph of trifluoroethane (HFC-143a), for a concentration of 5000 ppm HFC-143a. The column condenser is again counted as stage no. 1. The feed is introduced at stage 30 at temperature of -5 degrees C, with the column condenser pressure at 49.7 psia and the column base pressure 3 psi higher. The distillate temperature is -19.6 degrees C, and the bottom column temperature is about -16 to -18 degrees C. Under these conditions, the product HFC-125 leaves in the overhead (distillate) stream from the column. The remaining HFC-125 exits in the bottoms stream with HFC-143a as a waste product. The results of this example are shown in Table 13.

**Table 13**

| Reflux flow (lb/hr) | Reflux Ratio | Lb/hr HFC-125 in Bottoms | ppm HFC-143a in Distillate | % HFC-125 Recovery |
|---|---|---|---|---|
| 9379 | 11 | 144 | 100 | 85.6 |
| 9901 | 11 | 94 | 100 | 90.6 |
| 10710 | 11 | 43 | 100 | 95.7 |
| 14994 | 15 | 13 | 100 | 98.7 |
| 137000 | 138 | 4 | 729 | 99.6 |

While the goal of reducing the concentration of HFC-143a to 100 ppm HFC-143a is satisfied, the recovery of HFC-125 remains relatively low, even with relatively high reflux flows. The recovery efficiency is limited because an azeotropic or azeotrope-like composition is formed between HFC-125 and HFC-143a. However, this Example illustrates how these azeotrope compositions can be used to remove HFC-143a from an HFC-125 containing a mixture, e.g., thereby producing substantially pure HFC-125 by reducing the concentration of HFC-143a in the first mixture.

### Comparative Example 5

This Example is identical to Comparative Example 4, except that the feed stream to the column consists of 1000 pph HFC-125 and 52.63 pph HFC-143a, nominally 50,000 ppm HFC-143a, with the results shown in Table 14.

**Table 14**

| Reflux flow (lb/hr) | Reflux Ratio | Lb/hr HFC-125 in Bottoms | ppm HFC-143a in Distillate | % HFC-125 Recovery |
|---|---|---|---|---|
| 71241 | 78 | 86 | 100 | 91.4 |
| 206810 | 215 | 37 | 3730 | 96.3 |
| 341550 | 347 | 40 | 26649 | 98.3 |

This Example shows that even with extremely high reflux flows, the desired low concentration of HFC-143a in the product HFC-125 cannot be obtained without lowering the recovery efficiency of the product HFC-125. The recovery efficiency is limited because an azeotropic or azeotrope-like composition is formed between HFC-125 and HFC-143a. However, this Example illustrates how these azeotrope compositions can be used to remove HFC-143a from an HFC-125 containing mixture, e.g., thereby producing substantially pure HFC-125 by reducing the concentration of HFC-143a in the first mixture.

The above examples show that conventional distillation is not an economical way to separate HFC-143a from HFC-125, when substantially pure HFC-125 is desired, because such requires a relatively high reflux ratio, a large column diameter required to accommodate this high reflux ratio, high utilities cost for heating and cooling this large flow of material through the column, and the need for disposing of large quantities of by-product material containing HFC-143a. Such by-product material will also contain a relatively large quantity of HFC-125.

### Example 6

In this example, extractive distillation is used in a column with 67 stages for purifying a feed stream containing 1000 lb/hr of pentafluoroethane (HFC-125) and 5 lb/hr of HFC-143a or 4975 ppm as in Comparative Example 4. The pentafluoroethane stream to be purified is introduced onto column stage 50, and methanol is fed onto column stage 15. The feed stream is introduced at a temperature of 25 degrees C, and the methanol is introduced at a temperature -25 degrees C, with the column condenser pressure set at 19.7 psia and the column base pressure 3 psi higher. In general, the column pressures need be chosen only so that the temperature in the bottoms are below the critical temperatures of the individual components. The methanol is fed at varying rates as shown in Table 15, while the reflux flow is held at 5,000 lb/hr. The distillate temperature is -41 degrees C, and the bottom column temperature varies from about 38.0 to 69.1 degrees C.. The methanol and the HFC-125 exit through the bottom of the column, and the HFC-143a exits the top of the column.

In this and each example that follows, the methanol may be removed from the HFC-125 by using conventional distillation methods, e.g., a stripping column, or by scrubbing with water and drying with conventional methods such as a molecular sieve drier.

The compositions shown below in Table 15 are on a methanol-free basis.

**Table 15**

| Lb/hr Methanol Feed Rate | Lb/hr HFC-125 in Bottoms | ppm HFC-143a in Bottoms | % HFC-125 Recovery |
|---|---|---|---|
| 60000 | 1000 | 10 | 100 |
| 30000 | 1000 | 10 | 100 |
| 20000 | 1000 | 10 | 100 |
| 10000 | 1000 | 12 | 100 |

Example 6 shows that using methanol as an extractive agent permits recovering substantially pure HFC-125 at high yields while using a relatively small distillation column and extractant flow.

### Example 7

This Example is identical to Example 6, except that the feed stream consists of 1000 pph HFC-125 and 52.63 pph HFC-143a as in Comparative Example 5. The distillate temperature is still at -41 deg C, and the bottom column temperature varies from 68 to 70 deg c. The results of this Example are shown for two extractant flows as shown in Table 16.

**Table 16**

| Lb/hr Methanol Feed Rate | Lb/hr HFC-125 in Bottoms | ppm HFC-143a in Bottoms | % HFC-125 Recovery | ppm HFC-125 in Distillate |
|---|---|---|---|---|
| 70000 | 1000 | 10 | 100 | 0.57 |
| 60000 | 1000 | 10 | 100 | 1.90 |

Table 16 illustrates that in comparison to convention distillation, extractive distillation enhances the recovery rate and produces substantially pure HFC-125. The Example also illustrates that substantially pure HFC-143a can be recovered as an overhead product, and substantially pure HFC-125 can simultaneously be removed from the bottoms.

### Example 8

In this example, extractive distillation is used in a column with 57 stages for purifying a feed stream containing 990 lb/hr of pentafluoroethane (HFC-125), 5 lb/hr 1,1,1-trifluoroethane (HFC-143a), and 5 lb/hr chloropentafluoro-ethane (CFC-115). The pentafluoroethane containing stream to be purified is introduced onto column stage 40, and the extractant is fed onto column stage 15. The pentafluoroethane feed stream is introduced at a temperature of -20 degrees C, and the methanol is introduced at a temperature -20 degrees C. The column condenser pressure is set at 34.7 psia and the column base pressure is 3 psi higher. In general, the column pressures need be chosen only so that the temperature in the bottoms are below the critical temperatures of the individual components. The methanol is fed at varying rates as shown in Table 17, while the reflux flow is held at 5,000 lb/hr. The distillate temperature is -28.8 degrees C, and the bottom column temperature varies from about 67.9 to 76.7 degrees C. In this example, the methanol and HFC-125 exit through the bottom of the column, and the CFC-115 and the HFC-143a exit the top of the column.

The concentrations of CFC-115 and HFC-143a in the column bottoms shown in Table 17 are on a methanol-free basis.

**Table 17**

| Lb/hr Methanol Feed Rate | Lb/hr HFC-143a in Distillate | Lb/hr CFC-115 in Distillate | PPM Methanol in Distillate | PPM HFC-143a in Bottoms | PPM CFC-115 in Bottoms | Lb/hr HFC-125 in Bottoms |
|---|---|---|---|---|---|---|
| 25000 | 4.90 | 5.00 | <0.01 | 100 | <1.0 | 990 |
| 20000 | 4.90 | 5.00 | <0.01 | 100 | <1.0 | 990 |
| 15000 | 4.90 | 5.00 | <0.01 | 100 | <1.0 | 990 |

In this example, substantially 100 % of the HFC-125 fed in the feed mixture exits the column in the bottoms stream along with the methanol extractant, and CFC-115 and HFC-143a are removed in the column distillate.

### Example 9

In this example, extractive distillation is used in a column with 57 stages for purifying a feed stream containing 990 lb/hr of pentafluoroethane (HFC-125), 5 lb/hr 1,1,1-trifluoroethane (HFC-143a), and 5 lb/hr chloropentafluoro-ethane (CFC-115). The pentafluoroethane containing stream to be purified is introduced onto column stage 40, and the extractant is fed onto column stage 15. The pentafluoroethane feed stream is introduced at a temperature of -20 degrees C, and the methanol is introduced at a temperature 20 degrees C. The column condenser pressure is set at 34.7 psia and the column base pressure is 3 psi higher. In general, the column pressures need be chosen only so that the temperature in the bottoms are below the critical temperatures of the individual components. The methanol is fed at varying rates as shown in Table 18, while the reflux flow is held at 5,000 lb/hr. The distillate temperature is -18.1 degrees C, and the bottom column temperature varies from about 67.7 to 76.6 degrees C. In this example, the methanol, HFC-125 and HFC-143a exit through the bottom of the column, and the CFC-115 exits the top of the column.

The concentration of CFC-115 in the column bottoms shown in Table 18 is on a methanol-free basis.

**Table 18**

| Lb/hr Methanol Feed Rate | PPM HFC-143a in Distillate | Lb/hr CFC-115 in Distillate | PPM Methanol in Distillate | Lb/hr HFC-143a in Bottoms | PPM CFC-115 in Bottoms | Lb/hr HFC-125 in Bottoms |
|---|---|---|---|---|---|---|
| 25000 | <0.01 | 5.00 | <0.01 | 5.0 | <1.0 | 990 |
| 20000 | <0.01 | 5.00 | <0.01 | 5.0 | <1.0 | 990 |
| 15000 | <0.01 | 5.00 | <0.01 | 5.0 | <1.0 | 990 |

In this example, 100 % of the HFC-125 and HFC-143a present within the feed stream exits the column in the bottoms stream along with the methanol extractant. This Example shows that the relative volatility of CFC-115 and HFC-143a in the presence of methanol extractant is such that by adjusting the rate at which distillate is removed from the column, it is possible to preferentially remove CFC-115 overhead while removing HFC-143a, HFC-125 and methanol from the column bottoms, e.g., reducing the distillate rate in comparison to that described in Example 8. Consequently, it is possible, by adjusting the distillate removal rate, to selectively withdraw the HFC-143a either with the CFC-115 from the column distillate (as in Example 8) or with the HFC-125 and methanol from the column bottoms (as in Example 9).

### Example 10

In this example, extractive distillation is used in a column with 57 stages for purifying a feed stream containing 440 lb/hr of pentafluoroethane (HFC-125), 520 lb/hr 1,1,1-trifluoroethane (HFC-143a), 40 lb/hr 1,1,1,2-tetrafluoroethane (CF₃CH₂F, or HFC-134a) and 5.025 lb/hr of chloropentafluoroethane (CFC-115) or 5000 ppm CFC-115. The pentafluoroethane containing stream to be purified is introduced onto column stage 40, and the extractant is fed onto column stage 20. The pentafluoroethane feed stream is introduced at a temperature of -20 degrees C, and the methanol is introduced at a temperature -20 degrees C. The column condenser pressure is set at 34.7 psia and the column base pressure is 3 psi higher. In general, the column pressures need be chosen only so that the temperature in the bottoms are below the critical temperatures of the individual components. The methanol is fed at varying rates as shown in Table 19, while the reflux flow is held at 5,000 lb/hr. The distillate temperature is -18.1 degrees C, and the bottom column temperature varies from about 57.0 to 69.8 degrees C. The methanol, HFC-143a, HFC-134a and the HFC-125 exit through the bottom of the column, and the CFC-115 exits the top of the column.

The concentration of CFC-115 in the column bottoms shown in Table 19 is on a methanol-free basis.

**Table 19**

| Lb/hr Methanol Feed Rate | Lb/hr CFC-115 in Distillate | ppm HFC-143a in Distillate | ppm CFC-115 in Bottoms | Lb/hr Organic * in Bottoms |
|---|---|---|---|---|
| 25000 | 5.02 | 18 | 1.0 | 1000 |
| 20000 | 5.02 | 80 | 1.0 | 1000 |
| 15000 | 5.02 | 600 | 1.0 | 1000 |

| | | | | |
|---|---|---|---|---|
| *Organic = HFC-125, HFC-134a and HFC-143a. | | | | |

This example shows that extractive distillation is used to selectively remove CFC-115 from a HFC-125 containing blend. This example also shows that a mixture containing HFC-143a, HFC-134a and HFC-125 can be produced such that the mixture is substantially free of CFC-115.

### Example 11

In this example, extractive distillation is used in a column with 57 stages for purifying a feed stream containing 465 lb/hr of pentafluoroethane (HFC-125), 465 lb/hr 1,1,1-trifluoroethane (HFC-143a), 70 lb/hr chlorodifluoromethane (CHClF₂, or HCFC-22) and 5.025 Ib/hr of chloropentafluoroethane (CFC-115) or 5000 ppm CFC-115. The pentafluoroethane containing stream to be purified is introduced onto column stage 40, and the extractant is fed onto column stage 20. The pentafluoroethane feed stream is introduced at a temperature of -20 degrees C, and the methanol is introduced at a temperature -20 degrees C. The column condenser pressure is set at 34.7 psia and the column base pressure is 3 psi higher. In general, the column pressures need be chosen only so that the temperature in the bottoms are below the critical temperatures of the individual components. The methanol is fed at varying rates as shown in Table 20, while the reflux flow is held at 5,000 lb/hr. The distillate temperature is -18.1 degrees C, and the bottom column temperature varies from about 58.8 to 70.8 degrees C. The methanol, HFC-143a, HCFC-22 and the HFC-125 exit through the bottom of the column, and the CFC-115 exits the top of the column.

The concentration of CFC-115 in the column bottoms shown in Table 20 is on a methanol-free basis.

**Table 20**

| Lb/hr Methanol Feed Rate | Lb/hr CFC-115 in Distillate | ppm HFC-143a in Distillate | ppm CFC-115 in Bottoms | Lb/hr Organic * in Bottoms |
|---|---|---|---|---|
| 25000 | 5.02 | 16 | 1.0 | 1000 |
| 20000 | 5.02 | 60 | 1.0 | 1000 |
| 15000 | 5.02 | 600 | 1.0 | 1000 |

| | | | | |
|---|---|---|---|---|
| * Organic = HFC-125, HFC-134a and HFC-143a. | | | | |

This example shows that extractive distillation can be used to selectively remove CFC-115 from a HFC-125 containing blend. This example also shows that a mixture containing HFC-143a, HCFC-22 and HFC-125 can be produced such that the mixture is substantially free of CFC-115.

### Example 12

This Example demonstrates the existence of azeotropic and azeotrope-like compositions between the binary pair mixtures consisting essentially of HFC-125 and HFC-143a;

To determine the relative volatility of each binary pair, the so-called PTx Method was used. In this procedure, for each binary pair, the total absolute pressure in a PTx cell of known volume was measured at a constant temperature for various known binary compositions. These measurements were then reduced to equilibrium vapor and liquid compositions in the cell using the NRTL equation.

The vapor pressure measured versus the composition in the PTx cell for the HFC-125/HFC-143a system is shown in Figure 2. The experimental data points are shown in the Figure as solid points and the curve is drawn by using computer modeling.

Referring now to Figure 2, Fig. 2 illustrates graphically the formation of an azeotropic and azeotrope-like composition consisting essentially of HFC-125 and HFC-143a at -16.9 deg C, as indicated by a mixture of about 27.2 mole % HFC-125 and 72.8 mole % HFC-143a having the lowest pressure over the range of compositions at this temperature. Based upon these findings, it has been calculated that an azeotropic or azeotrope-like composition of about 41.1 mole % HFC-125 and 58.9 mole % HFC-143a is formed at -50 deg C and 12.5 psia and an azeotropic or azeotrope-like composition of about 5.2 mole % HFC-125 and 94.8 mole % HFC-143a is formed at 10 deg C and 122.1 psia. Accordingly, the present invention provides an azeotropic or azeotrope-like composition consisting essentially of from about 5 to about 41 mole % HFC-125 and from about 95 to about 59 mole % 143a, said composition having a boiling point of from about -50 deg C at 12 psia to about 10 deg C at 122 psia.

While particular emphasis has been placed upon using certain extractive agents, a mixed extractant may be effectively used in the inventive process. For example, by using a mixture of extractant agents the inventive process can be maximized to obtain HFC-125 that has the desired purity while minimizing the extractive agent cost. Alternatively, a series of one or more chemically similar or distinct extractive agents can be employed for maximizing the effectiveness of the inventive process. Further, the azeotrope distillation process illustrated above can also be used alone or in conjunction with the inventive extractive distillation process.

While certain aspects of the invention have been described in particular detail, a person in this art would understand that other embodiments and variations are covered by the appended claims.

## Claims

1. A process for separating pentafluoroethane from a first mixture comprising pentafluoroethane and at least one of 1,1,1-trifluoroethane and a chloropentafluoroethane, the process comprising the steps of:
adding at least one extractive agent comprised of an aliphatic alcohol or mixture of aliphatic alcohols having a boiling point at atmospheric pressure greater than about 64 degrees C and less than about 100 degrees C, to the first mixture in order to form a second mixture;
separating at least one of chloropentafluoroethane and 1,1,1-trifluoroethane from the second mixture by extractively distilling the second mixture in an extractive distillation zone and thereby separating said at least one of chloropentatluoroethane and 1,1,1-trifluoroethane and forming a third mixture comprising the extractive agent and pentafluoroethane and optionally 1,1,1-trifluoroethane; and
separating the pentafluoroethane from the third mixture to yield pentafluoroethane.

2. A process for separating 1,1,1-trifluoroethane from a first mixture comprising 1,1,1-trifluoroethane and pentafluoroethane, the process comprising the steps of :
adding at least one alcohol containing extractive agent having a boiling point at atmospheric pressure greater than about 64 degrees C, wherein said agent is substantially fluorine-free, to the first mixture in order to form a second mixture;
separating 1,1,1-trifluoroethane and pentafluoroethane by extractively distilling the second mixture in an extractive distillation zone and thereby separating 1,1,1-trifluoroethane and pentafluoroethane; and,
recovering at least one product from the group of 1,1,1-trifluoroethane and pentafluoroethane.

3. A process for separating a chlorofluorocarbon and/or 1,1,1-trifluoroethane from a first mixture comprising pentafluoroethane and at least one of a chlorofluorocarbon, 1,1,1-trifluoroethane and the process comprising the steps of:
adding at least one alcohol extractive agent having 1 to 4 carbon atoms and having a boiling point at atmospheric pressure of at least about 64°C to the first mixture in order to form a second mixture;
separating said at least one of chlorofluorocarbon and 1,1,1-trifluoroethane from the pentafluoroethane of the second mixture by extractively distilling the second mixture in an extractive distillation zone and thereby recovering said at least one of chlorofluorocarbon and 1,1,1-trifluoroethane as an overhead stream and forming a third mixture comprising the extractive agent and pentafluoroethane as a bottoms stream; and.
separating the extractive agent from the third mixture to yield a product from the group consisting of pentafluoroethane an pentafluoroethane with 1,1,1-trifluoroethane.

4. The process of claim 1 or 2 wherein the aliphatic alcohol has from one to four carbon atoms.

5. The process of claim 1, 2 or 3 wherein the extractive agent comprises at least one member selected from methanol, ethanol, n-propanol, isopropanol, sec-butanol and tert-butanol.

6. The process of claim 1, 2 or 3 wherein the extractive agent in admixture with pentafluoroethane is removed by distillation to yield substantially pure pentafluoroethane.

7. The process of claim 1, 2 or 3 wherein the extractive agent in admixture with pentafluoroethane is removed by extraction to yield substantially pure pentafluoroethane.

8. The process of claim I wherein the pentatluoroethane in the third mixture is scrubbed with water to remove said alcohol thereby producing substantially pure pentafluoroethane.

9. The method of claim 1 or 3 wherein said extractive distillation zone is within a distillation column and the third mixture is recovered from the bottom of the distillation column.

10. The method of claim 1, 2 or 3 wherein said extractive distillation zone is within a distillation column and the second mixture comprises chloropentafluoroethane which is recovered from the top of the distillation column.

11. The method of claim 1, 2 or 3 wherein the recovered pentafluoroethane is substantially pure.

12. The method of claim 1 or 2 wherein the second mixture comprises chloropentafluoroethane which is recovered as substantially free of pentafluoroethane.

13. The process of any one of claims 1, 2 or 3 further comprising distilling the first mixture prior to said adding at least one extractive agent.

14. The process of claim 13 wherein said distilling is performed under conditions sufficient to form an HFC-125 containing azeotropic or azeotrope-like composition.

15. The process of claim 14 wherein said azeotropic or azeotrope-like composition consists essentially of from 5 to 41 mole % HFC-125 and from 95 to 59 mole % HFC-143a, said composition having a boiling point of from -50 degrees C to 10 degrees C and a vapor pressure of from 12 to 122 psia.

16. The process of any one of claims 1, 2, 3 or 15 wherein the first mixture further comprises at least one of HFC-125, HFC-134a, HFC-143a and HCFC-22.

17. The process of claim 16 wherein the recovered product comprises HFC-143a, HFC-134a and HFC-125.

## Patentansprüche

1. Verfahren zum Trennen von Pentafluorethan aus einer ersten Mischung, umfassend Pentafluorethan und wenigstens eine Substanz von 1,1,1-Trifluorethan und einem Chlorpentafluorethan, wobei das Verfahren die Schritte umfaßt:
- Hinzufügen wenigstens eines Extraktionsagens, das einen aliphatischen Alkohol oder eine Mischung aliphatischer Alkohole mit einem Siedepunkt bei Atmosphärendruck von größer als etwa 64°C und weniger als etwa 100°C umfaßt, zu der ersten Mischung, um eine zweite Mischung zu bilden;
- Trennen wenigstens einer Substanz von Chlorpentafluorethan und 1,1,1-Trifluorethan aus der zweiten Mischung, indem die zweite Mischung in einer Extraktionsdestillationszone extraktiv destilliert wird und dadurch wenigstens eine Substanz von Chlorpentafluorethan und 1,1,1-Trifluorethan abgetrennt wird, und Erzeugen einer dritten Mischung, die das Extraktionsagens und Pentafluorethan und wahlweise 1,1,1-Trifluorethan umfaßt, sowie
- Trennen des Pentafluorethans von der dritten Mischung, um Pentafluorethan zu liefern.

2. Verfahren zum Trennen von 1,1,1-Trifluorethan aus einer ersten Mischung, die 1,1,1-Trifluorethan und Pentafluorethan umfaßt, wobei das Verfahren die Schritte umfaßt:
- Hinzufügen von wenigstens einem Alkohol, der ein Extraktionsagens mit einem Siedepunkt bei Atmosphärendruck von größer als etwa 64°C enthält, wobei das Agens im wesentlichen fluorfrei ist, zu der ersten Mischung, um eine zweite Mischung zu erzeugen;
- Trennen von 1,1,1-Trifluorethan und Pentafluorethan, indem die zweite Mischung in einer Extraktionsdestillationszone extraktiv destilliert wird und dadurch 1,1,1-Trifluorethan und Pentafluorethan abgetrennt werden; und
- Gewinnen von wenigstens einem Produkt aus der Gruppe von 1,1,1,-Trifluorethan und Pentafluorethan.

3. Verfahren zum Trennen von Chlorfluorkohlenstoff und/oder 1,1,1-Trifluorethan aus einer ersten Mischung, umfassend Pentafluorethan und wenigstens eine Substanz von Chlorfluorkohlenstoff, 1,1,1-Trifluorethan, wobei das Verfahren die Schritte umfaßt:
- Hinzufügen von wenigstens einem Extraktionsagens mit Alkohol mit 1 bis 4 Kohlenstoffatomen und mit einem Siedepunkt bei Atmosphärendruck von wenigstens etwa 64°C zu der ersten Mischung, um eine zweite Mischung zu erzeugen;
- Trennen von wenigstens einem von Chlorfluorkohlenstoff und 1,1,1-Trifluorethan von dem Pentafluorethan der zweiten Mischung, indem die zweite Mischung extraktiv in einer Extraktionsdestillationszone destilliert wird und dadurch wenigstens eines von den beiden, nämlich Chlorfluorkohlenstoff und 1,1,1-Trifluorethan, als ein Überkopfstrom gewonnen wird, und Erzeugen einer dritten Mischung, die das Extraktionsagens und Pentafluorethan als Sumpfstrom umfaßt, und
- Trennen des Extraktionsagens von der dritten Mischung, so daß ein Produkt aus der Gruppe, bestehend aus Pentafluorethan, einem Pentafluorethan mit 1,1,1-Trifluorethan, erzeugt wird.

4. Verfahren nach Anspruch 1 oder 2, wobei der aliphatische Alkohol 1 bis 4 Kohlenstoffatome aufweist.

5. Verfahren nach Anspruch 1, 2 oder 3, wobei das Extraktionsagens wenigstens einen Stoff enthält, der aus Methanol, Ethanol, n-Propanol, Isopropanol, sek.-Butanol und tert.-Butanol ausgewählt ist.

6. Verfahren nach Anspruch 1, 2 oder 3, wobei das Extraktionsagens in Mischung mit Pentafluorethan durch Destillation entfernt wird, um ein im wesentlichen reines Pentafluorethan zu erzeugen.

7. Verfahren nach Anspruch 1, 2 oder 3, wobei das Extraktionsagens in Mischung mit Pentafluorethan durch Extraktion entfernt wird, um ein im wesentlichen reines Pentafluorethan zu erzeugen.

8. Verfahren nach Anspruch 1, wobei das Pentafluorethan in der dritten Mischung mit Wasser gewaschen wird, um den Alkohol zu entfernen, wodurch ein im wesentlichen reines Pentafluorethan erzeugt wird.

9. Verfahren nach Anspruch 1 oder 3, wobei die Extraktionsdestillationszone sich innerhalb einer Destillationskolonne befindet und die dritte Mischung aus dem Sumpf der Destillationskolonne gewonnen wird.

10. Verfahren nach Anspruch 1, 2 oder 3, wobei die Extraktionsdestillationszone sich innerhalb einer Destillationskolonne befindet und die zweite Mischung Chlorpentafluorethan umfaßt, das am Kopf der Destillationskolonne gewonnen wird.

11. Verfahren nach Anspruch 1, 2 oder 3, wobei das gewonnene Pentafluorethan im wesentlichen rein ist.

12. Verfahren nach Anspruch 1 oder 2, wobei die zweite Mischung Chlorpentafluorethan umfaßt, das im wesentlichen frei von Pentafluorethan gewonnen wird.

13. Verfahren nach einem der Ansprüche 1, 2 oder 3, des weiteren umfassend, daß die erste Mischung destilliert wird, bevor das Hinzufügen von wenigstens einem Extraktionsagens durchgeführt wird.

14. Verfahren nach Anspruch 13, wobei das Destillieren unter Bedingungen durchgeführt wird, die dazu ausreichend sind, eine HFC-125-haltige azeotrope oder azeotropartige Zusammensetzung zu erzeugen.

15. Verfahren nach Anspruch 14, wobei die azeotrope oder azeotropartige Zusammensetzung im wesentlichen aus 5 bis 41 Molprozent HFC-125 und 95 bis 59 Molprozent HFC-143a besteht, wobei die Zusammensetzung einen Siedepunkt von -50°C bis 10°C sowie einen Dampfdruck von 12 bis 122 psia aufweist.

16. Verfahren nach einem der Ansprüche 1, 2, 3 oder 15, wobei die erste Mischung zusätzlich wenigstens eines von HFC-125, HFC-134a, HFC-143a und HCFC-22 umfaßt.

17. Verfahren nach Anspruch 16, wobei das gewonnene Produkt HFC-143a, HFC-134a und HFC-125 umfaßt.

## Revendications

1. Procédé pour séparer du pentafluoroéthane d'un premier mélange comprenant du pentafluoroéthane et au moins un composé parmi du 1,1,1-trifluoroéthane et du chloropentafluoroéthane, procédé comprenant les étapes de :
addition d'au moins un agent d'extraction comprenant un alcool aliphatique ou un mélange d'alcools aliphatiques ayant un point d'ébullition à pression atmosphérique supérieur à environ 64 degrés C et inférieur à environ 100 degrés C, au premier mélange pour former un deuxième mélange;
séparation d'au moins un composé parmi du chloropentafluoroéthane et du 1,1,1-trifluoroéthane du deuxième mélange en distillant de manière extractive le deuxième mélange dans une zone de distillation extractive et ainsi séparation dudit au moins un composé parmi du chloropentafluoroéthane et du 1,1,1-trifluoroéthane et formation d'un troisième mélange comprenant l'agent d'extraction et du pentafluoroéthane et facultativement du 1,1,1-trifluoroéthane, et
séparation du pentafluoroéthane du troisième mélange pour donner du pentafluoroéthane.

2. Procédé pour séparer du 1,1,1-trifluoroéthane d'un premier mélange comprenant du 1,1,1-trifluoroéthane et du pentafluoroéthane, le procédé comprenant les étapes de :
addition d'au moins un agent d'extraction contenant de l'alcool ayant un point d'ébullition à pression atmosphérique supérieur à environ 64 degrés C, dans lequel ledit agent est pratiquement exempt de fluor, au premier mélange afin de former un deuxième mélange.
séparation du 1,1,1-trifluoroéthane et du pentafluoroéthane par distillation de manière extractive du deuxième mélange dans une zone de distillation extractive et ainsi séparation du 1,1,1-trifluoroéthane et du pentafluoroéthane, et
récupération d'au moins un produit du groupe du 1,1,1-trifluoroéthane et du pentafluoroéthane.

3. Procédé pour séparer un chlorofluorocarbure et/ou du 1,1,1-trifluoroéthane d'un premier mélange comprenant du pentafluoroéthane et au moins un composé parmi un chlorofluorocarbure, du 1,1,1-trifluoroéthane et le procédé comprenant les étapes de :
addition d'au moins un agent d'extraction alcool ayant 1 à 4 atomes de carbone et ayant un point d'ébullition à pression atmosphérique d'au moins environ 64°C au premier mélange afin de former un deuxième mélange;
séparation d'au moins un composé parmi un chlorofluorocarbure et du 1,1,1-trifluoroéthane du pentafluoroéthane du deuxième mélange par distillation de manière extractive du deuxième mélange dans une zone d'extraction extractive et ainsi récupération dudit au moins un composé parmi un chlorofluorocarbure et du 1,1,1-trifluoroéthane comme flux de tête et formation d'un troisième mélange comprenant l'agent d'extraction et du pentafluoroéthane comme flux à la base, et
séparation de l'agent d'extraction du troisième mélange pour donner un produit du groupe constitué du pentafluoroéthane et du pentafluoroéthane avec du 1,1,1-trifluoroéthane.

4. Procédé suivant la revendication 1 ou 2, dans lequel l'alcool aliphatique a de un à quatre atomes de carbone.

5. Procédé suivant la revendication 1, 2 ou 3, dans lequel l'agent d'extraction comprend au moins un membre sélectionné parmi du méthanol, de l'éthanol, du n-propanol, de l'isopropanol, du sec-butanol et du tert-butanol.

6. Procédé suivant la revendication 1, 2 ou 3, dans lequel l'agent d'extraction mélangé à du pentafluoroéthane est éliminé par distillation pour donner du pentafluoroéthane pratiquement pur.

7. Procédé suivant la revendication 1, 2 ou 3, dans lequel l'agent d'extraction mélangé à du pentafluoroéthane est éliminé par extraction pour donner du pentafluoroéthane pratiquement pur.

8. Procédé suivant la revendication 1, dans lequel le pentafluoroéthane dans le troisième mélange est récuré à l'eau pour éliminer ledit alcool produisant ainsi du pentafluoroéthane pratiquement pur.

9. Procédé suivant la revendication 1 ou 3, dans lequel ladite zone de distillation extractive se trouve à l'intérieur d'une colonne de distillation et le troisième mélange est recueilli par la base de la colonne de distillation.

10. Procédé suivant la revendication 1, 2 ou 3, dans lequel ladite zone de distillation extractive se trouve à l'intérieur d'une colonne de distillation et le deuxième mélange comprend du chloropentafluoroéthane qui est recueilli par le haut de la colonne de distillation.

11. Procédé suivant la revendication 1, 2 ou 3, dans lequel le pentafluoroéthane recueilli est pratiquement pur.

12. Procédé suivant la revendication 1 ou 2, dans lequel le deuxième mélange comprend du chloropentafluoroéthane qui est recueilli pratiquement exempt de pentafluoroéthane.

13. Procédé suivant l'une quelconque des revendications 1, 2 ou 3, comprenant en outre la distillation du premier mélange avant ladite addition d'au moins un agent d'extraction.

14. Procédé suivant la revendication 13 dans lequel ladite distillation est réalisée dans des conditions suffisantes pour former un HCF-125 contenant une composition azéotropique ou de type azéotrope.

15. Procédé suivant la revendication 14 dans lequel la composition azéotropique ou de type azéotrope est constituée essentiellement de 5 à 41% en mole de HFC-125 et de 95 à 59% en mole de HFC-143a, ladite composition ayant un point d'ébullition de -50 degrés C à 10 degrés C et une pression vapeur de 12 à 1769 kPa (122 psia).

16. Procédé suivant l'une quelconque des revendications 1, 2, 3 ou 15, dans lequel le premier mélange comprend en outre au moins un composé parmi du HFC-125, du HFC-134a, du HFC-143a et du HCFC-22.

17. Procédé suivant la revendication 16 dans lequel le produit recueilli comprend du HFC-143a, du HFC-134a et du HFC-125.
